# EUROPEAN PATENT APPLICATION

(11) **EP 3 730 942 A1**
(43) Date of publication of application: **28.10.2020**
(21) Application number: 19382321.8
(22) Date of filing: 26.04.2019
(51) Int. Cl.: G01N 33/68

(54) **DIAGNOSIS MARKERS FOR ATRIAL FIBRILLATION**

(71) Applicant: Fundació Hospital Universitari Vall d'Hebron - Institut de Recerca, 08035 Barcelona (ES)
(72) Inventor: BUSTAMANTE RANGEL, Alejandro, 08013 BARCELONA (ES); PALÁ VILA, Elena, 08242 MANRESA (ES); MONTANER VILLALONGA, Joan, 08037 BARCELONA (ES)
(74) Representative: ZBM Patents - Zea, Barlocci & Markvardsen

(57) **Abstract**

The present application refers to an in vitro method for diagnosing atrial fibrilation in a subject, the method comprising determining in an isolated sample from the subject the levels of N-terminal fragment of B-type natriuretic peptide (NT-proBNP) and of at least one protein selected from the group consisting of R-spondin-3, Interleukin-1 receptor-like 1 isoform X1 (ST-2) and tissue inhibitor of metalloproteinases 2 (TIMP2), wherein when the levels of NT-proBNP and at of at least one protein selected from the group consisting of R-spondin-3, ST-2 and TIMP2 are higher than a reference value for each one this is indicative that the subject suffers from atrial fibrillation. The application also refers to method for recommending a medical regime for treating atrial fibrillation and/or for preventing and/or treating an atrial fibrillation associated condition in a subject based on this diagnosis, as well as to the use of the combined biomarkers for diagnosis of atrial fibrillation.

## Description

### Technical Field

The present invention is related to the field of biomarkers for diagnosis, in particular for diagnosis of atrial fibrillation.

### Background Art

Atrial fibrillation (AF) is a heart rhythm disorder that multiplies by five the risk of ischemic stroke. AF affects 3% of the general population and its prevalence increases with age. Anticoagulation may reduce stroke risk in AF individuals by approximately 60% but is associated with an increased risk of bleeding. As a result, in order to initiate preventive anticoagulation therapy, the risk of bleeding must be weighed against the benefits of the anticoagulation therapy, and documentation of atrial fibrillation is required.

Some guidelines recommend opportunistic screening for AF in patients >65 years in order to timely apply preventive anticoagulation therapy in subjects in need thereof. However, even if screening is performed, AF is often underdiagnosed, and consequently, untreated, because it sometimes courses as paroxysmal and asymptomatic. Paroxysmal AF are episodes of AF that occur occasionally and then stop spontaneously. Episodes can last for a few seconds or a few days before stopping and returning to normal sinus rhythm. Paroxysmal AF is frequently not detected with the use of traditional monitoring techniques such as in-hospital monitoring, serial electrocardiography (ECG). Different studies have tried to develop new AF systematic screening strategies, but none of them have yielded satisfactory results for clinical application.

Detecting paroxysmal AF is highly relevant to implement appropriate therapies for stroke prevention, but it is clinically challenging. Therefore new strategies are needed for detecting paroxysmal AF.

### Summary of Invention

The inventors have found an alternative method for the diagnosis of atrial fibrillation which is based on the combination of biomarkers. It was surprisingly found that when blood serum levels of N-terminal fragment of B-type natriuretic peptide (NT-proBNP) and at least one other marker selected from R-spondin-3 (RspO3), Interleukin-1 receptor-like 1 isoform X1 (ST-2) and tissue inhibitor of metalloproteinases 2 (TIMP2) are higher than a reference value for each one this is indicative of the patient having a higher risk of suffering from atrial fibrillation. The availability of reliable blood biomarkers for diagnosing atrial fibrillation has several advantages when compared to conventional diagnosis methods such as in-hospital monitoring, serial electrocardiography (ECG), long-term outpatient monitoring and Holter monitoring. Obtaining blood is easier than almost any other body fluid, and blood-based tests are amenable to high-throughput and cheap measurements. A blood-based marker could be obtained routinely in the community in primary care or even in the patient's home. Screening for AF in high risk population, or even in general population, is a far easier task when blood biomarkers having good discriminating ability (good sensibility and specificity) are available.

The present invention thus refers in a first aspect to an in vitro method for diagnosing atrial fibrillation in a subject, the method comprising determining in an isolated sample from the subject the levels of N-terminal fragment of B-type natriuretic peptide (NT-proBNP) and of at least one protein selected from the group consisting of R-spondin-3, and tissue inhibitor of metalloproteinases 2 (TIMP2), wherein when the levels of NT-proBNP and at of at least one protein selected from the group consisting of R-spondin-3, ST-2 and TIMP2 are higher than a reference value for each one this is indicative that the subject suffers from atrial fibrillation.

An important advantage of the present invention is that it allows for diagnosing paroxysmal AF with good sensibility and specificity, even in asymptomatic patients. Conventional methods for diagnosing AF do not diagnose the subjects with paroxysmal AF because they are asymptomatic, usually have less risk factors and heart monitoring (ECG and holter) is only done during a determined time frame. Occasional (paroxysmal) AF is simply missed out. Other biomarkers described in the prior art for diagnosis of AF may not have these drawbacks, but their predictive value is not good enough for detecting paroxysmal AF.

While providing for a reliable and early diagnosis of AF, for example, by being able to determine if a subject has paroxysmal AF, the present diagnosis method is useful to a clinician in the sense that the method enables him/her to take the most appropriate decisions to treat the patient. In particular, the present diagnosis allows the health professionals to recommend a therapeutic regime for the prevention of AF-associated disorders, for example ischemic stroke, or for the treatment of the atrial fibrillation. Based on the present diagnosis method, the health professional will usually recommend anticoagulation therapy to a subject that is diagnosed as suffering from AF with the aim of preventing an ischemic stroke. In particular, subjects at risk of suffering AF-associated disorders which would never be detected by other means can be included in preventive strategies, for example by administering anticoagulation therapy. The present diagnosis method is also useful to determine the cause of a stroke in patients having suffered from this pathology, for which the cause is unknown, and properly adjust the treatment regime for such patients.

In a second aspect the invention thus provides an in vitro method for recommending a medical regime for the treatment of AF and/or the prevention and/or treatment of an AF-associated condition in a subject, the method comprising a) diagnosing if the subject suffers from atrial fibrillation by the method as defined above, and b) recommending a medical regime for the treatment of AF and/or the prevention and/or treatment of an AF-associated condition if the subject is diagnosed of suffering from atrial fibrillation. The AF-associated condition is usually ischemic stroke and the recommended medical regime is usually anticoagulation therapy. Thus, the invention provides an in vitro method for recommending anticoagulation therapy in a subject, the method comprising a) diagnosing if the subject suffers from atrial fibrillation by the method as defined above, and b) recommending anticoagulation therapy if the subject is diagnosed of suffering from atrial fibrillation.

The diagnosis method of the invention may be provided in the form of a package or kit of parts. Usually said package or kit contains means for determining the amount of the disclosed biomarkers, together with instructions enabling the comparison of the results of said determination with reference values in order to make the diagnosis.

Another aspect of the invention thus provides a package or kit of parts for performing the diagnosis method defined above, comprising appropriate reagents for determining the amount of NT-proBNP and at least one protein selected from the group consisting of R-spondin-3, ST-2 and TIMP2 in a biological sample of a patient, and enabling the comparison of the combined results of said determination with reference values.

Another aspect of the invention provides for use of the N-terminal fragment of B-type natriuretic peptide (NT-proBNP) in combination with at least one protein selected from the group consisting of R-spondin-3, ST-2 and TIMP2 as biomarkers for the diagnosis of atrial fibrillation.

As mentioned above, diagnosis of AF, in particular, of difficult to detect paroxysmal AF, is very useful for general strategies for preventing AF-related conditions, in particular ischemic stroke. Therefore, a final aspect of the invention provides for use of NT-proBNP in combination with at least one protein selected from the group consisting of R-spondin-3, ST-2 and TIMP2 as biomarkers for ischemic stroke prevention.

### Brief Description of Drawings

**Figure 1****:** Subjects classification. Individuals were classified depending on AF presence and subgroups were made depending on past medical history (PMH) for AF, electrocardiogram findings (ECG) and Holter AF detection as follows: PMH-ECG-Holter+ or hAF (n=7), PMH-ECG+ (n=5), PMH+ECG- (n=2) and PMH+ECG+ (n=6)
**Figure 2****.** Box-plots of RspO3 circulating levels.
**Figure 3****.** Box-plots of STS circulating levels.

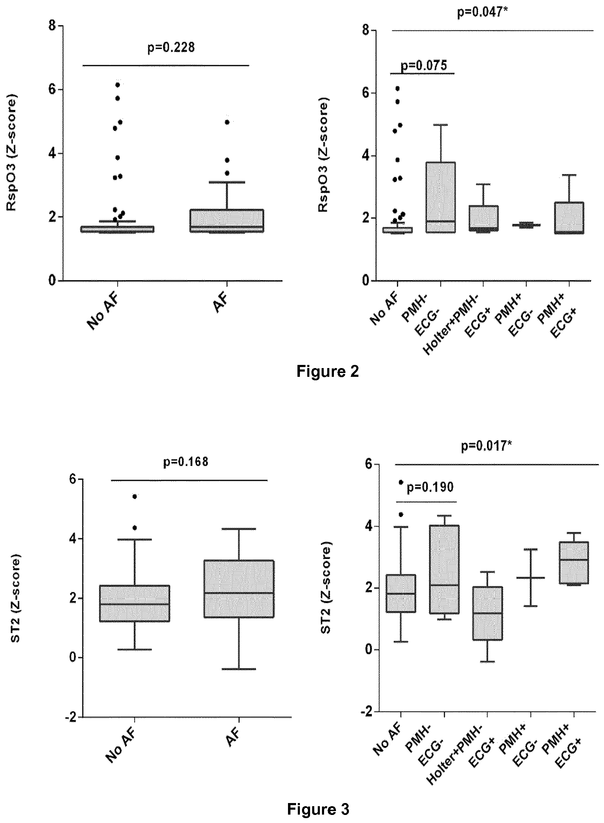

**Figure 3****.** Box-plots of TIMP2 circulating levels.

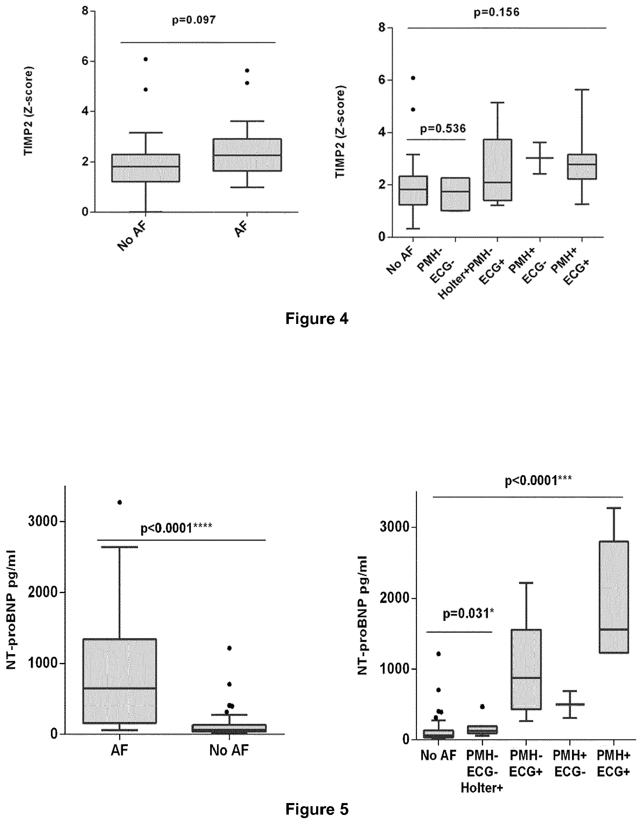

**Figure 5****.** Box-plots of NT-proBNP circulating levels

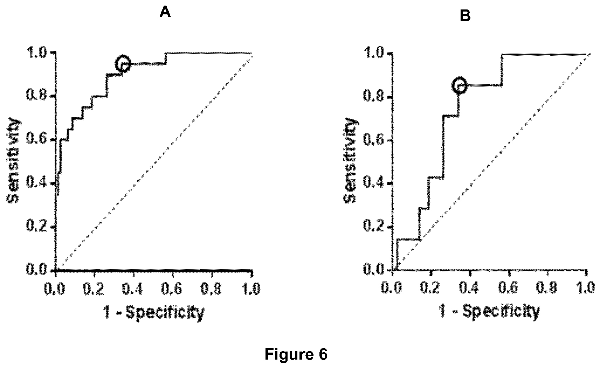

**Figure 6****:** ROC curve indicating NT-proBNP biomarker discrimination power. A, AF vs no AF. B, paroxysmal AF vs no AF.

### Detailed description of the invention

The term "diagnosis" is known to the person skilled in the art. As used herein "diagnosis" is understood as becoming aware of a particular medical condition, complication; the determination of the nature of the condition; or the distinguishing of the condition from another. It refers both to the process of attempting to determine or identify the possible condition, and to the opinion reached by this process. A diagnosis, in the sense of diagnostic procedure, can be regarded as an attempt at classification of an individual's condition into separate and distinct categories that allow medical decisions about treatment and prognosis to be made.

The term "reference value" in the context of the present invention is to be understood as a predefined level of the biomarker in a sample or group of samples. The samples are taken from a subject or group of subjects wherein the presence, absence, stage, or course of the disease has been properly performed previously. This value is used as a threshold to discriminate subjects wherein the condition to be analysed is present from those wherein such condition is absent. The skilled person in the art, making use of the general knowledge, is able to choose the subject or group of subjects more adequate for obtaining the reference value. Methods for obtaining the reference value from the group of subjects selected are well known in the state of the art (Burtis C. A. et al., 2008, Chapter 14, section "Statistical Treatment of Reference Values"). In a particular case the "reference value" is a cut-off value defined by means of a conventional ROC analysis (Receiver Operating Characteristic analysis). As will be apparent to the skilled person the reference values will be defined according to the purpose, target population for the diagnosis, balance between specificity and sensibility, etc.

The present invention requires comparing the levels of the biomarkers in the biological sample extracted from the subject with a reference value. In the sense of the present invention, the expression "higher than a reference value" is understood as any increase in the level of protein.

The term "immunochemistry" as used herein refers to a variety of techniques for detecting antigens (in the present case any of the proteins encoded by the above genes or antigenic fragments thereof) in a sample by exploiting the principle of antibodies binding specifically to the target protein(s). Visualizing an antibody-antigen interaction can be then accomplished in a number of ways, usually by conjugating the antibody to an enzyme, such as peroxidase, that can catalyse a colour-producing reaction, or to a fluorophore, such as fluorescein or rhodamine. The immunochemistry technique can be direct or indirect.

The term "antibody or a fragment thereof able to bind to the target protein(s)" is to be understood as any immunoglobulin or fragment thereof able to selectively bind the target protein(s) referred in the aspects and embodiments of the present invention. It includes monoclonal and polyclonal antibodies. The term "fragment thereof" encompasses any part of an antibody having the size and conformation suitable to bind an epitope of the target protein. Suitable fragments include F(ab), F(ab') and Fv. An "epitope" is the part of the antigen being recognized by the immune system (B-cells, T-cells or antibodies).

The N-terminal fragment of B-type natriuretic peptide (NT-proBNP) (SEQ ID NO: 1) is the 76-amino acid N-terminal fragment of the B-type natriuretic peptide prohormone. Cleaving of pro-BNP yields the NT-proBNP fragment and the active B-type natriuretic peptide (BNP). BNP is a hormone secreted by cardiomyocytes in the heart ventricles in response to stretching caused by increased ventricular blood volume.

R-spondin-3 (RspO3) (ncbi reference number NP_116173.2, SEQ ID NO: 2) is a protein that in humans is encoded by the RSPO3 gene. The protein contains a furin-like cysteine-rich region. Furin-like repeat domains have been found in a variety of eukaryotic proteins involved in the mechanism of signal transduction by receptor tyrosine kinases. During embryonic development, RSPO3 is expressed in the tail bud and the posterior presomitic mesoderm of the embryo. In tissue engineering, R-spondin 3 has been used to differentiate pluripotent stem cells into paraxial mesoderm progenitors.

Interleukin-1 receptor-like 1 isoform X1 (ST-2) (ncbi reference number XP_011510453.1, SEQ ID NO: 3) is a member of the interleukin 1 receptor family

Tissue inhibitor of metalloproteinases 2 (TIMP2) (ncbi reference number NP_003246.1, SEQ ID NO: 4) is a protein thought to be a metastasis suppressor. TIMP2 complexes with metalloproteinases (such as collagenases) and irreversibly inactivates them by binding to their catalytic zinc cofactor.

The present disclosure relates to a method for diagnosing AF by determining the levels of NT-proBNP and at least one of R-spondin-3, ST-2 and TIMP2 in a sample obtained from a subject.

Blood NT-proBNP has a high predictive accuracy for AF. The predictive accuracy (area under ROC curve) of NT-proBNP was found to be 0.900 (IC 95%, 0.8274-0.9726, p<0.0001) to detect any AF (Figure 6 A). A cut-off point of NT-proBNP >95pg/ml showed 95% sensitivity and 66.2% specificity to detect any AF. The present disclosure thus also refers to an in vitro method for diagnosing atrial fibrillation in a subject, the method comprising determining in an isolated sample from the subject the levels of N-terminal fragment of B-type natriuretic peptide (NT-proBNP), wherein when the levels are higher than a reference value this is indicative that the subject suffers from atrial fibrillation.

Moreover, the inventors have now found that this biomarker (NT-proBNP) may also be detected in blood samples of subjects suffering from paroxysmal AF. The predictive accuracy of NT-proBNP was 0.7464 (IC 95% 0.6087-0.8841, p=0.031) to detect paroxysmal AF (Figure 6 B). The same cut-off point of NT-proBNP >95pg/ml showed 85.7 % sensitivity, 65% specificity to detect paroxysmal AF.

In one embodiment the levels of NT-proBNP and R-spondin-3 are determined. In another embodiment, the levels of NT-proBNP and ST-2 may be determined. In another example, the levels of NT-proBNP and TIMP2 are determined. In another embodiment, the levels of NT-proBNP and at least two proteins selected from the group consisting of R-spondin-3, ST-2 and TIMP2 are determined. For example, the levels of NT-proBNP, R-spondin-3 and ST-2 are determined. In another example, the levels of NT-proBNP, R-spondin-3 and TIMP2 are determined. In another example, the levels of NT-proBNP, ST-2 and TIMP2 are determined.

In another embodiment, the levels of all four biomarkers (NT-proBNP, R-spondin-3, ST-2 and TIMP2) are determined.

The predictive value of the combination of biomarkers is significantly better than that of NT-proBNP on its own. In particular, the predictive value of a combination of biomarkers including NT-proBNP and two out of R-spondin-3, ST-2 and TIMP2 has a predictive accuracy (area under the ROC curve) of 85.4 (64.3-100.0), with 85.7% sensitivity and 90.3% specificity for paroxysomal AF detection. Such a high predictive value for detecting AF has not been disclosed so far.

Many of the AF patients that may be newly diagnosed with the method of the invention correspond to those having paroxysmal or asymptomatic AF. In one embodiment, the method of the invention is for diagnosing paroxysmal or asymptomatic atrial fibrillation. In particular the method of the invention is for diagnosing paroxysmal AF.

In some embodiments the subjects have not suffered from stroke. In some embodiments the subjects are asymptomatic for AF. In other embodiments the subjects have suffered from a previous stroke, particularly ischemic stroke.

The biological sample isolated from the subject may be any tissue, or a bodily fluid such as blood, plasma, saliva, urine, cerebrospinal fluid, or semen. However, in one preferred embodiment of the invention the biological sample is blood, particularly peripheral blood. This is important because it greatly speeds up and simplifies the detection method, plus it is non-invasive. In another particular embodiment the sample is blood serum. Usually, a blood sample is extracted from the subject and centrifuged to obtain blood serum, which is subsequently used for determining the levels of the biomarkers.

Reference values have been determined for the biomarkers of the invention. The reference value for NT-proBNP may be from 50 to 150 pg/ml. In another embodiment the reference value for NT-proBNP may be from 50 to 123 pg/ml. For example, a NT-proBNP reference value from 68-123 pg/ml provides for diagnosis of AF with particularly high sensibilitiy and specificity. Thus, in one embodiment, the reference value of NT-proBNP is from 68 to 123 pg/ml, or from 81 to 110 pg/ml pg protein/ml, or from 90 to 100 pg protein/ml, for example 95 pg protein/ml. The reference value for R-spondin-3 may be from 50 to 700 pg/ml. For example, a R-spondin-3 reference value from 123 to 563 pg protein/ml provides for diagnosis of AF with particularly high sensibility and specificity. Thus, in one embodiment, the reference value of R-spondin-3 is from 80 to 450 pg/ml, or from 100 to 300 pg/ml, or from 100 to 200 pg protein/ml, for example 155 pg protein/ml. The reference value for ST-2 may be from 10 to 60 ng protein/ml. In another embodiment the reference value for ST-2 may be from 20 to 60 ng protein/ml. For example, a reference value for ST-2 from 34 to 44 ng protein/ml provides for diagnosis of AF with particularly high sensibility and specificity. Thus, in one embodiment, the reference value of ST-2 is from 10 to 60 ng protein/ml, or from 34 to 44 ng protein/ml, for example 40 ng protein/ml. The reference value for TIMP2 may be from 50 to 200 ng protein/ml. For example, a reference value for TIMP2 from 66 to 79 ng protein/ml provides for diagnosis of AF with particularly high sensibility and specificity. Thus, in one embodiment, the reference value of TIMP2 is from 60 to 90 ng protein/ml, or from 66 to 79, for example 72. In a particular embodiment, the reference values are, independently, from 68 to 123 pg/ml for NT-proBNP, from 123 to 563 pg protein/ml for R-spondin-3, from 34 to 44 ng protein/ml for ST-2 and from 66 to 79 ng protein/ml for TIMP2.

In some embodiments, the method of the invention is adjusted to provide the best sensibility in order to avoid false negatives such that every single subject suffering from AF is identified. In one example this high sensibility is obtained by determining the levels of NT-proNTP, ST-2 and TIMP-2 (see example 2 below). The reference values for the biomarkers in this case may be, independently, from 50 to 65 pg/ml for NT-proBNP, from 10 to 30 ng protein/ml for ST-2 and from 100 to 200 ng protein/ml for TIMP2. In particular, the reference values may be, independently, from 55 to 60 pg/ml for NT-proBNP, from 15 to 20 ng protein/ml for ST-2 and from 140 to 170 ng protein/ml for TIMP2, for example, from 57 pg/ml for NT-proBNP, 18 ng protein/ml for ST-2 and 158 ng protein/ml for TIMP2.

In a further particular application of the present diagnosis method, AF may be determined in patients who have suffered from a stroke, but for which the cause of the stroke is unknown. In this case, the levels of NT-proNTP and at least one or more of Rspo3, ST-2 and TIMP-2 are determined and the reference value for NT-proNTP may be from 100 to 150 pg/ml, or from 120 to 130 pg/ml.

The levels of the protein biomarkers of the invention are determined by a quantitative test selected from the group consisting of an immunological test, bioluminescence, fluorescence, chemiluminescence, electrochemistry and mass spectrometry.

In one embodiment the level of encoded protein or fragment thereof is detected by mass spectrometry, for example, by Shotgun Liquid Chromatography Mass Spectrometry (LC-MS/MS) or Multiple reaction monitoring (MRM) mass spectrometry.

In an alternative embodiment, the level of expression is determined by immunochemistry. Suitable immunoassay procedures include enzyme-linked immunosorbent assays (ELISA, such as multiplex ELISA), enzyme immunodot assay, agglutination assay, antibody-antigen-antibody sandwich assay, antigen-antibody-antigen sandwich assay, immunocromatography, or other immunoassay formats well-known to the ordinarily skilled artisan, such as radioimmunoassay, as well as protein microarray formats. In one embodiment, the level of the protein is determined by an immunoassay. In another embodiment, the level of expression of protein is determined by ELISA.

Screening for AF in risk populations is recommended by some stroke prevention guidelines in order to detect patients that would benefit from anticoagulation therapy. Risk factors to be considered are known by the skilled person and may help determine the study population. The present diagnosis method is particularly suitable for said screening strategies. Thus, in particular embodiments, the method as defined above is performed in subjects having AF-associated risk factors or stroke-associated risk factors. For example, the subjects selected for performing the method as defined above are those suffering from a condition selected from one or more of hypertension, old age and diabetes. That is, the risk-factors may be hypertension, diabetes and/or age older than 65.

Screening in risk populations is often recommended because of the impossibility to perform the screening in the general population. This is because the cost of the diagnosis methods usually do not compensate for the low incidence of a particular disease in the general population. However, screening for AF with blood biomarkers as disclosed herein is a feasible strategy that might be considered for screening general population, or in aged population (such as over 65 years), in particular because the present diagnosis method is able to detect asymptomatic or paroxysmal AF with high accuracy. Therefore, in particular embodiments the subject of the method of the invention does not necessarily have an AF risk-associated factor, that is, he/she forms part of the general population or is simply an elderly subject, for example over 65 years. In particular, the subject is asymptomatic for atrial fibrillation.

Further, the diagnosis method of the invention as defined may be performed in addition to conventional methods for diagnosing AF. Said conventional methods include in a non-limitative manner, monitoring techniques such as in-hospital monitoring, serial electrocardiography (ECG), long-term outpatient monitoring and Holter monitoring. Conventional methods may also include determining at least one clinical parameter, wherein preferably said clinical parameter is selected from the group consisting of age, sex, presence of vascular risk factors, medications, smoking, presence of diabetes mellitus, presence of previous stroke and presence of hypertension.

In a second aspect the invention provides an in vitro method for recommending a medical regime for the prevention and/or treatment of AF or an AF-associated condition in a subject in a subject by diagnosing if the subject suffers from atrial fibrillation by the method as defined above and recommending a medical regime for prevention and/or treatment of AF or an AF-associated condition if he/she does.

Medical regimes for treating AF and for preventing and/or treating atrial fibrillation-associated conditions are known in the art. The most common atrial fibrillation-associated condition is ischemic stroke. Thus in one embodiment the method is for recommending a medical regime for preventing stroke, in particular ischemic stroke. Usually, the diagnosis of AF will lead the medical professional to recommend a medical regime comprising anticoagulants for preventing ischemic stroke. Sometimes the recommended medical regime comprises antiplatelet drugs (also called antiaggregants). Sometimes the treatment comprises both anticoagulants and antiplatelets.

Antiplatelet and anticoagulant therapies play a vital role in stroke treatment and prevention. While both groups of medications prevent the formation of a clot, each works on a different clot etiology and subsequently alters different intrinsic pathways. Different antiplatelet agents inhibit different receptors on platelets. While oral anticoagulants prevent the function of various clotting factors such as factors II, VII, IX, and X. Antiplatelet agents have strong clinical evidence supporting their use for prevention of noncardioembolic stroke, whereas anticoagulants are strongly recommended for prevention of most types of cardioembolic stroke.

In certain embodiments, the anticoagulation therapy to be recommended when the subject is diagnosed with AF by the method described above includes recommending any of the commonly used anticoagulants, for example, warfarin, acenocumarol, dabigatran, rivaroxaban, apixaban, and edoxaban. In other embodiments, the anticoagulation therapy to be recommended when the subject is diagnosed with AF by the method described above includes recommending any of the commonly used antiplatelets, such as aspirin, clopidogrel, and Aggrenox, or combinations thereof.

The diagnosis method of the invention may be provided in the form of a package or kit of parts containing appropriate reagents for determining the amount of the biomarkers disclosed above and preferably means or instructions for enabling the comparison of the combined results of said determination with reference values. The means or instructions for enabling the comparison of the combined results of said determination with reference values may include the reference values or instructions as to how to calculate said reference values. The reagents may be an antibody or a fragment thereof able to bind to the target protein, labels and other reagents to perform an immunoassay, for example, an ELISA test. The reagents may comprise, for example, an immunochromatographic strip that will show a positive result when the level of the biomarker is above the reference value. The analytical technique to determine the level of biomarker is not limited and therefore neither are the reagents to perform said technique.

In one embodiment the package or kit of parts contains reagents to determine the level of NT-proBNP. In another embodiment the package or kit of parts contains reagents to determine the level of NT-proBNP and at least one other biomarker selected from R-spondin-3, ST-2 and TIMP2. In another embodiment, the package or kit of parts contains reagents to determine the level of NT-proBNP and at least two other biomarkers selected from R-spondin-3, ST-2 and TIMP2. In another embodiment, the package or kit of parts contains reagents to determine the level of NT-proBNP, R-spondin-3, ST-2 and TIMP2.

The invention also provides for the use of NT-proBNP in combination with at least one protein selected from the group consisting of R-spondin-3, ST-2 and TIMP2 as biomarkers for the diagnosis of atrial fibrillation. In one embodiment, the invention provides for the use of NT-proBNP in combination with at least two proteins selected from the group consisting of R-spondin-3, ST-2 and TIMP2 as biomarkers for the diagnosis of atrial fibrillation. In another embodiment, the invention provides for the use of NT-proBNP in combination with R-spondin-3, ST-2 and TIMP2 as biomarkers for the diagnosis of atrial fibrillation. In particular embodiments the biomarkers are used for diagnosing paroxysmal atrial fibrillation. In other embodiments the biomarkers are used for recommending a treatment regime for the treatment of AF and/or for the prevention or treatment of AF-associated disorders, such as ischemic stroke. In particular, the biomarkers are used for recommending anticoagulation therapy to a subject in need thereof.

The diagnosis method of the invention may be automated in order to provide a diagnosis result. Thus, the invention also provides a system for diagnosing AF in a subject comprising data processing means, said data processing means been configured:
- to assess in a biological sample obtained from the subject the level of NT-proBNP and at least one other protein selected from R-spondin-3, ST-2 and TIMP2;
- to determine whether said levels of NT-proBNP and at least one other protein selected from R-spondin-3, ST-2 and TIMP2, are above predetermined reference values for each protein; and
- to diagnose if the patient suffers from AF by evaluating the result of the previous determination.

The in vitro methods of the invention generally provide for diagnosis of AF and for recommending an appropriate therapy, for example, anticoagulant therapy. In some embodiments said methods may further comprise the steps of (i) collecting the diagnostic information, and (ii) saving the information in a data carrier.

In the sense of the invention a "data carrier" is to be understood as any means that contain meaningful information data for the diagnosis of AF and/or recommendation of an appropriate therapy, such as paper. The carrier may also be any entity or device capable of carrying the AF diagnosis data or information for recommending an appropriate therapy. For example, the carrier may comprise a storage medium, such as a ROM, for example a CD ROM or a semiconductor ROM, or a magnetic recording medium, for example a floppy disc or hard disk. Further, the carrier may be a transmissible carrier such as an electrical or optical signal, which may be conveyed via electrical or optical cable or by radio or other means. When the diagnosis data are embodied in a signal that may be conveyed directly by a cable or other device or means, the carrier may be constituted by such cable or other device or means. Other carriers relate to USB devices and computer archives. Examples of suitable data carrier are paper, CDs, USB, computer archives in PCs, or sound registration with the same information.

The present invention also discloses the following detailed embodiments:
Embodiment 1. An in vitro method for diagnosing atrial fibrillation in a subject, the method comprising determining in an isolated sample from the subject the level of N-terminal fragment of B-type natriuretic peptide (NT-proBNP), wherein when the level of NT-proBNP is higher than a reference value this is indicative that the subject suffers from atrial fibrilation.
Embodiment 2. An in vitro method for diagnosing atrial fibrillation in a subject, the method comprising determining in an isolated sample from the subject the levels of N-terminal fragment of B-type natriuretic peptide (NT-proBNP) and of at least one protein selected from the group consisting of R-spondin-3, Interleukin-1 receptor-like 1 isoform X1 (ST-2) and tissue inhibitor of metalloproteinases 2 (TIMP2), wherein when the levels of NT-proBNP and at of at least one protein selected from the group consisting of R-spondin-3, ST-2 and TIMP2 are higher than a reference value for each one this is indicative that the subject suffers from atrial fibrillation.
Embodiment 3. The in vitro method according to embodiment 2, wherein the levels of NT-proBNP and R-spondin-3 are determined.
Embodiment 4. The in vitro method according to embodiment 2, wherein the levels of NT-proBNP and ST-2 are determined.
Embodiment 5. The in vitro method according to embodiment 2, wherein the levels of NT-proBNP and TIMP2 are determined.
Embodiment 6. The in vitro method according to embodiment 2, wherein the levels of NT-proBNP, R-spondin-3 and ST-2 are determined.
Embodiment 7. The in vitro method according to embodiment 2, wherein the levels of NT-proBNP, ST-2 and TIMP2 are determined.
Embodiment 8. The in vitro method according to any of the embodiments 1-7, that is for diagnosing paroxysmal atrial fibrillation.
Embodiment 9. The in vitro method according to any of the embodiments 1-8, that is for diagnosing asymptomatic atrial fibrillation.
Embodiment 10. The in vitro method according to any of the embodiments 1-9, wherein the subject has suffered from a stroke.
Embodiment 11. The in vitro method according to any of the embodiments 1-9, wherein the subject has not suffered from a stroke.
Embodiment 12. The in vitro method according to any of the embodiments 1-11, wherein determining the levels of the proteins is performed by a quantitative test selected from the group consisting of an immunological test, bioluminescence, fluorescence, chemiluminescence, electrochemistry and mass spectrometry.
Embodiment 13. The in vitro method according to embodiment 12, wherein determining the levels of the proteins is performed by an immunoassay.
Embodiment 14. The in vitro method according to embodiment 13, wherein determining the levels of the proteins is performed by an ELISA.
Embodiment 15. The in vitro method according to embodiment 13, wherein determining the levels of the proteins is performed by immunochromatography.
Embodiment 16. The in vitro method according to any of the embodiments 1-15, wherein the sample is tissue, or a bodily fluid such as blood, plasma, saliva, urine, cerebrospinal fluid, or semen.
Embodiment 17. The in vitro method according to embodiment 16, wherein the sample is a blood sample or a plasma sample.
Embodiment 18. The in vitro method according to embodiment 17, wherein the reference values are, independently, from 50 to 150 pg protein/ml for NT-proBNP, from 50 to 700 pg protein/ml for R-spondin-3, from 20 to 60 ng protein/ml for ST-2 and from 50 to 200 ng protein/ml for TIMP2.
Embodiment 19. The in vitro method according to embodiment 18, wherein the reference values are, independently, from 68-123 pg/ml for NT-proBNP, from 123 to 563 pg protein/ml for R-spondin-3, from 34 to 44 ng/ml for ST-2 and from 66 to 79 ng/ml for TIMP2.
Embodiment 20. The in vitro method according to embodiment 19, wherein the reference values are, independently, 95 pg protein/ml for NT-proBNP, 155 pg protein/ml for R-spondin-3, 40 ng/ml for ST-2 and 72 ng/ml for TIMP2.
Embodiment 21. The in vitro method according to embodiment 17, wherein the reference values are, independently, from 50 to 65 pg/ml for NT-proBNP, from 10 to 30 ng protein/ml for ST-2 and from 100 to 200 ng protein/ml for TIMP2.
Embodiment 22. The in vitro method according to embodiment 21, wherein the reference values are, independently, from 55 to 60 pg/ml for NT-proBNP, from 15 to 20 ng protein/ml for ST-2 and from 140 to 170 ng protein/ml for TIMP2.
Embodiment 23. The in vitro method according to embodiment 17, wherein the reference value for NT-proBNP is from 100 to 150 pg/ml.
Embodiment 24. The in vitro method according to embodiment 23, wherein the reference value for NT-proBNP is from 120 to 130 pg/ml.
Embodiment 25. The in vitro method according to embodiment 17, wherein the reference value for NT-proBNP is from 50 to 123 pg/ml.
Embodiment 26. The in vitro method according to any of the embodiments 1-25, that additionally comprises use of conventional methods for diagnosing AF, said conventional methods being selected from in-hospital monitoring, serial electrocardiography (ECG), long-term outpatient monitoring, Holter monitoring, determining age, sex, presence of vascular risk factors, medications, smoking, presence of diabetes mellitus, presence of previous stroke and presence of hypertension.
Embodiment 27. The in vitro method according to any of the embodiments 1-26, wherein the subject is asymptomatic for atrial fibrillation.
Embodiment 28. The in vitro method according to any of the embodiments 1-26, wherein the subject has suffered from stroke.
Embodiment 29. The in vitro method according to any of the embodiments 1-28, wherein the subject suffers from a condition selected from hypertension, age older than 65, diabetes and combinations thereof.
Embodiment 30. An in vitro method for recommending a medical regime for the treatment of AF and/or for the prevention and/or treatment of an AF-associated condition in a subject, the method comprising
   a) diagnosing if the subject suffers from atrial fibrillation by the method as defined in any of the embodiments 1-29, and
   b) recommending a medical regime for the treatment of AF and/or for the prevention and/or treatment of an AF-associated condition if the subject is diagnosed of suffering from atrial fibrillation.
Embodiment 31. The in vitro method according to embodiment 30, wherein the medical regime is for preventing stroke.
Embodiment 32. The in vitro method according to any of the embodiments 30-31, wherein the medical regime comprises anticoagulation therapy.
Embodiment 33. The in vitro method according to embodiment 32, wherein the anticoagulation therapy is selected from the group consisting of warfarin, dabigatran, rivaroxaban, apixaban, and edoxaban, aspirin, clopidogrel, Aggrenox, and combinations thereof.
Embodiment 34. The in vitro method according to any of the embodiments 30-31, wherein the medical regime comprises antiplatelet therapy.
Embodiment 35. A package or kit of parts for performing the method defined in anyone of embodiments 1-34, comprising appropriate reagents for determining the amount of NT-proBNP and enabling the comparison of the result of said determination with a reference value.
Embodiment 36. A package or kit of parts for performing the method defined in anyone of embodiments 2-35, comprising appropriate reagents for determining the amount of NT-proBNP alone or in combination with and at least one protein selected from the group consisting of R-spondin-3, ST-2 and TIMP2 in a biological sample of a patient, and enabling the comparison of the combined results of said determination with reference values.
Embodiment 37. The package or kit of parts according to embodiment 36 that comprises reagents for determining the levels of NT-proBNP and R-spondin-3
Embodiment 38. The package or kit of parts according to embodiment 36 that comprises reagents for determining the levels of NT-proBNP and ST-2.
Embodiment 39. The package or kit of parts according to embodiment 36 that comprises reagents for determining the levels of NT-proBNP and TIMP2.
Embodiment 40. The package or kit of parts according to embodiment 36 that comprises reagents for determining the levels of NT-proBNP, R-spondin-3 and ST-2.
Embodiment 41. The package or kit of parts according to embodiment 36 that comprises reagents for determining the levels of NT-proBNP, ST-2 and TIMP2.
Embodiment 42. The package or kit of parts according to embodiment 36 that comprises reagents for determining the levels of NT-proBNP, R-spondin-3, ST-2 and TIMP2.
Embodiment 43. The package or kit of parts according to any of the embodiments 35-42, that comprises the reference values for the biomarkers.
Embodiment 44. The package or kit of parts according to any of the embodiments 35-43, wherein the reagents are for performing an immunoassay, for example an ELISA test.
Embodiment 45. The package or kit of parts according to embodiment 44, wherein the reagents comprise an antibody or a fragment thereof able to bind to the target protein.
Embodiment 46. The package or kit of parts according to embodiment 44, wherein the reagents comprise an immunochromatographic strip.
Embodiment 47. Use of the N-terminal fragment of B-type natriuretic peptide (NT-proBNP) as biomarker for the diagnosis of atrial fibrillation.
Embodiment 48. Use of R-spondin-3 as biomarker for the diagnosis of atrial fibrillation.
Embodiment 49. Use of ST-2 as biomarker for the diagnosis of atrial fibrillation.
Embodiment 50. Use of TIMP2 as biomarker for the diagnosis of atrial fibrillation.
Embodiment 51. The use according to embodiment 47, wherein NT-proBNP is combined with R-spondin-3.
Embodiment 52. The use according to embodiment 47, wherein NT-proBNP is combined with ST-2.
Embodiment 53. The use according to embodiment 47, wherein NT-proBNP is combined with TIMP2.
Embodiment 54. The use according to embodiment 47, wherein NT-proBNP is combined with R-spondin-3 and ST-2.
Embodiment 55. The use according to embodiment 47, wherein NT-proBNP is combined with ST-2 and TIMP2.
Embodiment 56. The use according to embodiment 47, wherein NT-proBNP is combined with R-spondin-3, ST-2 and TIMP2.
Embodiment 57. The use according to any of the embodiments 47-56, that is for diagnosing paroxysmal atrial fibrillation.
Embodiment 58. The use according to any of the embodiments 47-57, that is for diagnosing atrial fibrillation in asymptomatic subjects.
Embodiment 59. Use of NT-proBNP as biomarker for ischemic stroke prevention.
Embodiment 60. Use of R-spondin-3 as biomarker for ischemic stroke prevention.
Embodiment 61. Use of ST-2 as biomarker for ischemic stroke prevention.
Embodiment 62. Use of TIMP2 as biomarker for ischemic stroke prevention.
Embodiment 63. The use according to embodiment 59, wherein NT-proBNP is combined with R-spondin-3.
Embodiment 64. The use according to embodiment 59, wherein NT-proBNP is combined with ST-2.
Embodiment 65. The use according to embodiment 59, wherein NT-proBNP is combined with TIMP2.
Embodiment 66. The use according to embodiment 59, wherein NT-proBNP is combined with R-spondin-3 and ST-2.
Embodiment 67. The use according to embodiment 59, wherein NT-proBNP is combined with ST-2 and TIMP2.
Embodiment 68. The use according to embodiment 59, wherein NT-proBNP is combined with R-spondin-3, ST-2 and TIMP2.
Embodiment 69. A system for diagnosing atrial fibrillation in a subject comprising data processing means, said data processing means been configured:
   - to assess in a biological sample obtained from the subject the level of NT-proBNP;
   - to determine whether said levels of NT-proBNP are above a predetermined reference value; and
   - to diagnose if the patient suffers from AF by evaluating the result of the previous determination.
Embodiment 70. A system for diagnosing atrial fibrillation in a subject comprising data processing means, said data processing means being configured:
   - to assess in a biological sample obtained from the subject the levels of NT-proBNP and at least one other protein selected from R-spondin-3, ST-2 and TIMP2;
   - to determine whether said levels of NT-proBNP and at least one other protein selected from R-spondin-3, ST-2 and TIMP2, are above predetermined reference values for each one; and
   - to diagnose if the patient suffers from AF by evaluating the result of the previous determination.
Embodiment 71. The system according to embodiment 70, wherein the levels of NT-proBNP and R-spondin-3 are determined.
Embodiment 72. The system according to embodiment 70, wherein the levels of NT-proBNP and ST-2 are determined.
Embodiment 73. The system according to embodiment 70, wherein the levels of NT-proBNP and TIMP2 are determined.
Embodiment 74. The system according to embodiment 70, wherein the levels of NT-proBNP, R-spondin-3 and ST-2 are determined.
Embodiment 75. The system according to embodiment 70, wherein the levels of NT-proBNP, ST-2 and TIMP2 are determined.
Embodiment 76. The system according to any of the embodiments 69-75, that is for diagnosing paroxysmal atrial fibrillation.
Embodiment 77. The system according to any of the embodiments 69-76, that is for diagnosing asymptomatic atrial fibrillation.
Embodiment 78. The system according to any of the embodiments, wherein the sample is tissue, or a bodily fluid such as blood, plasma, saliva, urine, cerebrospinal fluid, or semen.
Embodiment 79. The system according to embodiment 78, wherein the sample is a blood sample or a plasma sample.
Embodiment 80. The system according to embodiment 79, wherein the reference values are, independently, from 50 to 150 pg protein/ml for NT-proBNP, from 50 to 700 pg protein/ml for R-spondin-3, from 20 to 60 ng protein/ml for ST-2 and from 50 to 200 ng protein/ml for TIMP2.
Embodiment 81. The system according to embodiment 80, wherein the reference values are, independently, from 68-123 pg/ml for NT-proBNP, from 123 to 563 pg protein/ml for R-spondin-3, from 34 to 44 ng/ml for ST-2 and from 66 to 79 ng/ml for TIMP2.
Embodiment 82. The system according to embodiment 81, wherein the reference values are, independently, 95 pg protein/ml for NT-proBNP, 155 pg protein/ml for R-spondin-3, 40 ng/ml for ST-2 and 72 ng/ml for TIMP2.

Unless otherwise stated, all terms as used herein in this application shall be understood in their ordinary meaning as known in the art. Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular and preferred embodiments and examples described herein.

### Examples

### Patients and methods

### Study population

The study population included asymptomatic subjects aged 65-75 with hypertension and diabetes selected from primary care registers. Participants were ambulatory patients in baseline situation, meaning they were contacted at home to arrange a visit in their primary care centre specifically for the study. During a single appointment, patients received a comprehensive assessment consisting of clinical characteristics (demographic factors, vascular risk factors, medications, comorbidities and vital signs), electrographic assessment, and a blood sample collection. Individuals with chronic inflammatory diseases, cancer or dementia were excluded. Patient's heart rhythm was monitored during four weeks with a wearable Holter device (Nuubo™, https://www.nuubo.com/). AF was defined as irregularly irregular R-R intervals without P wave signal, lasting for more than 60 seconds.

### Blood samples and biomarkers determination

Blood was collected into EDTA and Serum tubes at the time of inclusion. After centrifugation at 1,500 g and 4°C for 15 minutes, plasma and serum aliquots were frozen at -80°C until analysis.

First, the SOMAscan proteomic assay was performed by SomaLogic Inc (Boulder, CO, USA) in plasma samples from 26 subjects: 13 without AF and 13 with AF. SOMAscan is an aptamer-based proteomics assay capable of measuring 1,305 human protein analytes simultaneously.

A panel including 17 biomarkers was selected to test in the whole cohort: Interleukin-1 Receptor Like 1 (ST2), Tissue Inhibitor of Matrix Metalloproteinases 2 (TIMP-2), Chemokine (C-C motif) ligand 3-like 1 (CCL3L1), dermatopontin (DPT), Interleukin-1 Receptor A (IL-1RA), beta endorphin (POMC), Coagulation Factor IX (FIX), A disintegrin-like and metalloprotease with thrombospondin type 1 motif no. 13 (ADAMTS13), Bone Morphogenic Protein 1 (BMP-1), Polymeric immunoglobulin receptor (plgR) and interleukin 36 alpha (IL-36A). Other biomarkers were chosen from literature and previous results of our group, including NT-proBNP, von Willebrand factor (vWF), Apolipoprotein C3 (ApoC-III), R-spondin 3 (RspO3), Urokinase and Low affinity immunoglobulin gamma Fc region receptor II (FcgRII). Some of the biomarkers were chosen from a previous discovery study.

Plasma NT-proBNP levels were determined by automated immunoassay in a COBAS c8000 (Roche Diagnostics). The other biomarkers were measured by ELISA immunoassays as listed in Table 1.

**Table 1. Methods of biomarker measurement**

| **Biomaker** | **REF** | **Manufacturer** | **Blood type** | **Dilution** |
|---|---|---|---|---|
| **hST2/IL-1 R4** | DTS200 | R&D Systems | SERUM | 1/2 |
| **hTIMP-2** | DTM200 | R&D Systems | EDTA | 1/50 |
| **IL-1ra/IL-1F3** | DRA00B | R&D Systems | SERUM | 1/2 |
| **Adamts13 +vWF** | LXSAHM-02 | R&D Systems | EDTA | 1/2 |
| **Urokinase** | SEA140Hu | Cloud-clone | EDTA | 1/5 |
| **Fcg-RII** | SEB579Hu | Cloud-clone | SERUM | 1/100 |
| **APOC3** | KA0465 | Abnova | EDTA | 1/40 00 |
| **IX(F9)** | 00943 | Stago | EDTA | 1/102 |
| **POMC** | MBS2885899 | MyBiosource | EDTA | 1/2 |
| **RSPO3** | EK1512 | Boster | SERUM | pure |
| **IL36A** | MBS9428008 | MyBiosource | EDTA | 1/2 |
| **BMP1** | E-EL-H0585 | Elabscience | EDTA | 1/10 |
| **PIgR** | E-EL-H1236 | Elabscience | SERUM | 1/200000 |
| **CCL3L1** | E-EL-H0702 | Elabscience | SERUM | 1/2 |
| **Dermatopontin** | E-EL-H1802 | Elabscience | EDTA | 1/2 |

Each sample was assayed twice and the mean value of both measurements was used. All samples with a coefficient of variation (CV) between duplicates higher than 20% were excluded from the analysis. Samples with values under the detectable range of the assay were given the limit of detection value, whereas values over the detectable range were given the highest readable value. Inter-assay variation was determined by a commercial control (Human Serum, male AB, USA origin from clotted, SIGMA, ref number H16914/ Human plasma K2 EDTA, Innovative Research, ref number IPLA-N) tested in duplicated in each plate. Inter-assay variations over 20 were corrected using a Z-score.

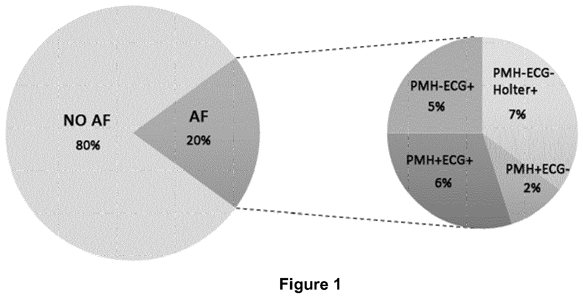

Of the 100 subjects included, AF was present in 20 (12 newly detected within the present study). Subjects were classified and compared in five groups depending on past medical history (PMH) for AF, ECG findings and Holter AF detection as follows: PMH-ECG-Holter+ or hAF (n=7), PMH-ECG+ (n=5), PMH+ECG- (n=2) and PMH+ECG+ (n=6) (Figure 1). Demographic and clinical information of these patients is shown in Table 2.

**Table 2. Clinical characteristics of the cohort and comparison according to atrial fibrillation diagnosis.**

| | **All patients (100)** | **AF (20)** | **No AF (80)** | **hAF (7)** | **P value¹** | **P value²** |
|---|---|---|---|---|---|---|
| **Age** | 70 (68-73) | 69 (66-71.5) | 70 (68-73) | 70 (65-72) | 0.273 | 0.655 |
| **Sex (% female)** | 33 (33) | 7 (35) | 26 (32.5) | 3 (42.9) | 0.832 | 0.682 |
| **Tobacco** | 20 (20) | 4 (20) | 16 (20.3) | 1 (14.3) | 1.00 | 1.000 |
| **Alcohol** | 11 (11) | 1 (5) | 10 (12.5) | 0 (0) | 0.456 | 1.000 |
| **Dislipidemia** | 81 (81) | 16 (80) | 65 (81.3) | 5 (71.4) | 1.00 | 0.619 |
| **Coronary heart disease** | 18 (18) | 8 (40) | 10 (12.5) | 3 (42.9) | 0.008* | 0.032* |
| **Heart failure** | 3 (3) | 3 (15) | 0 (0) | 0 (0) | 0.007* | 1.000 |
| **Valvular disease** | 4 (4) | 3 (15) | 1 (1.3) | 0 (0) | 0.024* | 0.767 |
| **Previous stroke** | 6 (6) | 2 (10) | 4 (5) | 0 (0) | 0.597 | 1.000 |
| **Anticoagulation** | 9 (9) | 8 (40) | 1 (1.3) | 0 (0) | 0.000* | 0.920 |
| **Antiplatelets** | 50 (50) | 7 (35) | 43 (53.8) | 3 (42,9) | 0.134 | 0.702 |
| **Familiar history FA** | 5 (5) | 2 (10) | 3 (3.9) | 0 (0) | 0.273 | 1.000 |
| **SBP, mm Hg** | 143.50 (134-153) | 140.5 (127.5-162.5) | 144 (134-151.75) | 140 (124-168) | 0.973 | 0.773 |
| **DBP, mm Hg** | 78.95±10.09 | 80.95±10.85 | 78.45±9.90 | 79.14±6.66 | 0.332 | 0.857 |
| **Heart rate** | 77.94±15.70 | 77.25±16.94 | 78.11±15.48 | 73±22.14 | 0.724 | 0.421 |
| **CHA2DS2-VASc** | 4 (3-4) | 4 (3-4) | 4 (3-5) | 4 (3-5) | 0.035 | 0.284 |

| | | | | | | |
|---|---|---|---|---|---|---|
| ¹Pvalue comparison AF vs no AF; ²Pvalue comparison hAF vs no AF; *Pvalue<0.05; AF: atrial fibrillation; DBP: dyastolic blood pressure; hAF:Holter-detected atrial fibrillation; SBP: systolic blood pressure | | | | | | |

### Results

The results of all the biomarkers determined are shown in table 3.

**Table 3. Biomarkers results.**

| | **AF** | **AFholter** | **NoAF** | **Pvalue¹** | **Pvalue²** |
|---|---|---|---|---|---|
| **ADAMTS13 ng/mL** | 1131.24 ± 471.42 | 1271.68 ± 561.01 | 1221.41 ± 459.81 | 0,437 | 0,786 |
| **vWF ng/mL** | 0.14 ± 0.53 | 0.15 ± 0.060 | 0.14 ± 0.07 | 0,987 | 0,802 |
| **ApoCIII ng/mL** | 107594 (92501-129262) | 105352 (91956-132268) | 104450 (76664-130393) | 0.496 | 0.574 |
| **Beta-endorphin (Z-score)** | 1.75 (1.46-2.25) | 1.73 (1.41-2.02) | 1.77 (1.53-2.14) | 0,868 | 0,744 |
| **BMP1 ng/mL** | 99.78 (81.82-104.54) | 85.20 (77.78-102.02) | 97.21 (86.23-104.51) | 0.930 | 0.220 |
| **CCL3L1 (Z-score)** | 1.81 (1.48-2.14) | 1.60 (1.41-2.02) | 1.71(1.53-2.15) | 0.596 | 0.501 |
| **DPT (Z-score)** | 1.76 (1.55-1.92) | 1.61 (1.47-1.84) | 1.71(1.58-2.15) | 0.813 | 0.360 |
| **FcgRII (Z-score)** | 2.54 (1.37-2.94) | 2.36 (1.00-2.82) | 1.87 (1.07-2.76) | 0.121 | 0.707 |
| **FIX %** | 118.50 ± 28.00 | 136.31 ± 15.55 | 133.52 ± 22.19 | 0.012 | 0.746 |
| **IL-1RA (Z-score)** | 1.77 (1.35-1.93) | 1.88 (1.69-1.94) | 1.71 (1.48-2.14) | 0.607 | 0.653 |
| **IL36A (Z-score)** | 1.74 ± 0.81 | 1.67 ± 0.87 | 2.07 ± 1.033 | 0.177 | 0.301 |
| **PIGR (Z-score)** | 1.99 (1.57-2.76) | 1.85 (1.26-2.20) | 1.73 (1.45-2.23) | 0.264 | 0.984 |
| **RSPO3 (Z-score)** | 1.69 (1.55-2.00) | 1.89 (1.55-3.79) | 1.55 (1.55-1.69) | 0.228 | 0.075 |
| **ST2 (Z-score)** | 2.26 ± 1.21 | 2.42 ± 1.32 | 1.91 ± 0.93 | 0.168 | 0.190 |
| **TIMP2 (Z-score**) | 2.26 (1.60- 2.84) | 1.73 (0.99-2.26) | 1.82(1.23-2.32) | 0.097 | 0.492 |
| **Urokinase (Z-score)** | 1.82 (1.41-2.39) | 1.68 (1.29-2.36) | 1.60 (1.38-2.35) | 0.720 | 0.868 |
| **NT-proBNP pg/mL** | 128.3 (97.69-191.3) | 643.65 (155.72-1339.25) | 64.28 (37.08-133.10) | 0.000 | 0.031 |

Four of the assayed biomarkers showed predictive value for the diagnosis of AF, namely, NT-proBNP, R-spondin-3, ST-2 and TIMP2.

Rspo3 was increased in paroxysmal AF individuals without previous AF history or detection with conventional ECG (p=0.075, Figure 2).

ST2 distribution was different between all groups (p=0.017) with a trend to be increased in AF individuals (0.168, Figure 3).

TIMP2 was increased in AF individuals (p=0.097, Figure 4)

NT-proBNP was increased in AF individuals (p=0.0001) even when only taking into account paroxysmal AF (p=0.031, Figure 5).

Discriminating ability (area under ROC curve) of NT-proBNP was 0.900 (IC 95%, 0.8274-0.9726, p<0.0001) to detect any AF (Fig6). A cut-off point of NT-proBNP >95pg/ml showed 95% sensitivity and 66.2% specificity to detect any AF. Discriminating ability (area under ROC curve) of NT-proBNP was 0.7464 (IC 95% 0.6087-0.8841, p=0.031) to detect paroxysmal AF (Figure 6). The same cut-off point of NT-proBNP >95pg/ml showed 85.7 % sensitivity, 65% specificity to detect paroxysmal AF.

The combination of the assayed biomarkers with the online PanelomiX tool (https://www.panelomix.net/) identified a 4-biomarker panel containing NT-proBNP, R-spondin-3, ST-2 and TIMP2. which provides good rates of sensitivity and specificity for AF diagnosis in high-risk subjects in which AF is not detected in a conventional ECG.

**Table 4.**

| | **PANEL. Positive whenever 2 markers, one of them being NT-proBNP, are positive** | | | |
|---|---|---|---|---|
| Biomarkers | R-spondin-3 | ST-2 | TIMP-2 | NT-proBNP |
| Direction | > | > | < | > |
| Reference value | 155.5 pg/mL | 40393.3 pg/mL | 72.7 ng/mL | 95 pg/mL |

It was found that when 2 out of the 4 biomarkers of this panel, one of them being NT-proBNP, is positive (above the reference value), the predictive accuracy was 85.4 (64.3-100.0), with 85.7% sensitivity and 90.3% specificity for AF detection.

An extensive study on the sensibility and specificity of the above panel was performed and the following reference value intervals were considered to provide particularly good sensibility and specificity for AF diagnosis.

**Table 5.**

| | Cut-off interval | Specificity interval | Sensibility interval |
|---|---|---|---|
| ST2 (pg/ml) | 34956.6-44171 | 85.5%-95.2% | 85.7%-71.4% |
| Rspo3 (pg/ml) | 123-563.9 | 90.3%-90.3% | 85.7%-85.7% |
| NT_proBNP (pg/ml) | 68.7-123.7 | 88.7%-93.5% | 85.7%-71.4% |
| TIMP2 (ng/ml) | 66.6-79.8 | 91.9%-88.7% | 85.7%-85.7% |

### EXAMPLE 2:

This example refers to the same population and biomarkers tan the previous example, but in this case the combination of the assayed biomarkers with the online PanelomiX tool (https://www.panelomix.net/) is performed looking for 100% sensitivity, in order to avoid any false negative. In this case, a 3-biomarker panel containing NT-proBNP, ST-2 and TIMP-2 was identified. It provided 100% sensitivity and 58.1% specificity for AF diagnosis in high-risk subjects in which AF is not detected in a conventional ECG.

| | **PANEL. Positive whenever any 3 markers are positive** | | |
|---|---|---|---|
| Biomarkers | ST-2 | TIMP-2 | NT-proBNP |
| Direction | > | < | > |
| Reference value | 18073.75 pg/mL | 158.88 ng/mL | 57.79 pg/mL |

### Citation List

Burtis C. A. et al., 2008, Chapter 14, section "Statistical Treatment of Reference Values" Nuubo™, https://www.nuubo.com/

## Claims

1. An in vitro method for diagnosing atrial fibrillation in a subject, the method comprising determining in an isolated sample from the subject the levels of N-terminal fragment of B-type natriuretic peptide (NT-proBNP) and of at least one protein selected from the group consisting of R-spondin-3, Interleukin-1 receptor-like 1 isoform X1 (ST-2) and tissue inhibitor of metalloproteinases 2 (TIMP2), wherein when the levels of NT-proBNP and at of at least one protein selected from the group consisting of R-spondin-3, ST-2 and TIMP2 are higher than a reference value for each one this is indicative that the subject suffers from atrial fibrillation.

2. The in vitro method according to claim 1, wherein the levels of at least two proteins selected from the group consisting of R-spondin-3, ST-2 and TIMP2 are determined.

3. The in vitro method according to claim 2, wherein the levels of NT-proBNP, R-spondin-3, ST-2 and TIMP2 are determined.

4. The in vitro method according to any of the claims 1-3, that is for diagnosing paroxysmal.

5. The in vitro method according to any of the claims 1-4, wherein the sample is a blood sample or a plasma sample.

6. The in vitro method according to claim 5, wherein the reference value for NT-proBNP is from 50-150 pg/ml.

7. The in vitro method according to any of the claims 1-6, wherein the subject is asymptomatic for atrial fibrillation.

8. The in vitro method according to any of the claims 1-7, wherein the subject suffers from a condition selected from hypertension, sleep apnoea syndrome, atherosclerosis and diabetes.

9. An in vitro method for recommending a medical regime for treating atrial fibrillation and/or for preventing and/or treating an atrial fibrillation associated condition in a subject, the method comprising
a) diagnosing if the subject suffers from atrial fibrillation by the method as defined in any of the claims 1-8, and
b) recommending a medical regime for treating atrial fibrillation and/or for preventing and/or treating an atrial fibrillation associated condition if the subject is diagnosed of suffering from atrial fibrillation.

10. The in vitro method according to claim 9, wherein the medical regime comprises administering anticoagulants.

11. A package or kit of parts for performing the method defined in anyone of claims 1 to 10, comprising appropriate reagents for determining the amount of NT-proBNP and at least one protein selected from the group consisting of R-spondin-3, ST-2 and TIMP2 in a biological sample of a patient, and enabling the comparison of the combined results of said determination with reference values.

12. Use of the N-terminal fragment of B-type natriuretic peptide (NT-proBNP) in combination with at least one protein selected from the group consisting of R-spondin-3, ST-2 and TIMP2 as biomarkers for the diagnosis of atrial fibrillation.

13. The use according to claim 12, wherein NT-proBNP is combined with at least two proteins selected from the group consisting of R-spondin-3, ST-2 and TIMP2

14. The use according to any of the claims 12-13, that is for diagnosing paroxysmal atrial fibrillation.

15. Use of NT-proBNP in combination with at least one protein selected from the group consisting of R-spondin-3, ST-2 and TIMP2 as biomarkers for ischemic stroke prevention.


SEQUENCE LISTING

<110>  FUNDACIÓ HOSPITAL UNIVERSITARI VALL D'HEBRON - INSTITUT DE
       RECERCA

<120>  Diagnosis markers for atrial fibrilation

<130>  P4954EP00

<160>  4

<170>  PatentIn version 3.5

<210>  1
<211>  76
<212>  PRT
<213>  Homo sapiens

<400>  1

His Pro Leu Gly Ser Pro Gly Ser Ala Ser Asp Leu Glu Thr Ser Gly
1               5                   10                  15

Leu Gln Glu Gln Arg Asn His Leu Gln Gly Lys Leu Ser Glu Leu Gln
            20                  25                  30

Val Glu Gln Thr Ser Leu Glu Pro Leu Gln Glu Ser Pro Arg Pro Thr
        35                  40                  45

Gly Val Trp Lys Ser Arg Glu Val Ala Thr Glu Gly Ile Arg Gly His
    50                  55                  60

Arg Lys Met Val Leu Tyr Thr Leu Arg Ala Pro Arg
65                  70                  75

<210>  2
<211>  272
<212>  PRT
<213>  Homo sapiens

<400>  2

Met His Leu Arg Leu Ile Ser Trp Leu Phe Ile Ile Leu Asn Phe Met
1               5                   10                  15

Glu Tyr Ile Gly Ser Gln Asn Ala Ser Arg Gly Arg Arg Gln Arg Arg
            20                  25                  30

Met His Pro Asn Val Ser Gln Gly Cys Gln Gly Gly Cys Ala Thr Cys
        35                  40                  45

Ser Asp Tyr Asn Gly Cys Leu Ser Cys Lys Pro Arg Leu Phe Phe Ala
    50                  55                  60

Leu Glu Arg Ile Gly Met Lys Gln Ile Gly Val Cys Leu Ser Ser Cys
65                  70                  75                  80

Pro Ser Gly Tyr Tyr Gly Thr Arg Tyr Pro Asp Ile Asn Lys Cys Thr
                85                  90                  95

Lys Cys Lys Ala Asp Cys Asp Thr Cys Phe Asn Lys Asn Phe Cys Thr
            100                 105                 110

Lys Cys Lys Ser Gly Phe Tyr Leu His Leu Gly Lys Cys Leu Asp Asn
        115                 120                 125

Cys Pro Glu Gly Leu Glu Ala Asn Asn His Thr Met Glu Cys Val Ser
    130                 135                 140

Ile Val His Cys Glu Val Ser Glu Trp Asn Pro Trp Ser Pro Cys Thr
145                 150                 155                 160

Lys Lys Gly Lys Thr Cys Gly Phe Lys Arg Gly Thr Glu Thr Arg Val
                165                 170                 175

Arg Glu Ile Ile Gln His Pro Ser Ala Lys Gly Asn Leu Cys Pro Pro
            180                 185                 190

Thr Asn Glu Thr Arg Lys Cys Thr Val Gln Arg Lys Lys Cys Gln Lys
        195                 200                 205

Gly Glu Arg Gly Lys Lys Gly Arg Glu Arg Lys Arg Lys Lys Pro Asn
    210                 215                 220

Lys Gly Glu Ser Lys Glu Ala Ile Pro Asp Ser Lys Ser Leu Glu Ser
225                 230                 235                 240

Ser Lys Glu Ile Pro Glu Gln Arg Glu Asn Lys Gln Gln Gln Lys Lys
                245                 250                 255

Arg Lys Val Gln Asp Lys Gln Lys Ser Val Ser Val Ser Thr Val His
            260                 265                 270

<210>  3
<211>  328
<212>  PRT
<213>  Homo sapiens

<400>  3

Met Gly Phe Trp Ile Leu Ala Ile Leu Thr Ile Leu Met Tyr Ser Thr
1               5                   10                  15

Ala Ala Lys Phe Ser Lys Gln Ser Trp Gly Leu Glu Asn Glu Ala Leu
            20                  25                  30

Ile Val Arg Cys Pro Arg Gln Gly Lys Pro Ser Tyr Thr Val Asp Trp
        35                  40                  45

Tyr Tyr Ser Gln Thr Asn Lys Ser Ile Pro Thr Gln Glu Arg Asn Arg
    50                  55                  60

Val Phe Ala Ser Gly Gln Leu Leu Lys Phe Leu Pro Ala Ala Val Ala
65                  70                  75                  80

Asp Ser Gly Ile Tyr Thr Cys Ile Val Arg Ser Pro Thr Phe Asn Arg
                85                  90                  95

Thr Gly Tyr Ala Asn Val Thr Ile Tyr Lys Lys Gln Ser Asp Cys Asn
            100                 105                 110

Val Pro Asp Tyr Leu Met Tyr Ser Thr Val Ser Gly Ser Glu Lys Asn
        115                 120                 125

Ser Lys Ile Tyr Cys Pro Thr Ile Asp Leu Tyr Asn Trp Thr Ala Pro
    130                 135                 140

Leu Glu Trp Phe Lys Asn Cys Gln Ala Leu Gln Gly Ser Arg Tyr Arg
145                 150                 155                 160

Ala His Lys Ser Phe Leu Val Ile Asp Asn Val Met Thr Glu Asp Ala
                165                 170                 175

Gly Asp Tyr Thr Cys Lys Phe Ile His Asn Glu Asn Gly Ala Asn Tyr
            180                 185                 190

Ser Val Thr Ala Thr Arg Ser Phe Thr Val Lys Asp Glu Gln Gly Phe
        195                 200                 205

Ser Leu Phe Pro Val Ile Gly Ala Pro Ala Gln Asn Glu Ile Lys Glu
    210                 215                 220

Val Glu Ile Gly Lys Asn Ala Asn Leu Thr Cys Ser Ala Cys Phe Gly
225                 230                 235                 240

Lys Gly Thr Gln Phe Leu Ala Ala Val Leu Trp Gln Leu Asn Gly Thr
                245                 250                 255

Lys Ile Thr Asp Phe Gly Glu Pro Arg Ile Gln Gln Glu Glu Gly Gln
            260                 265                 270

Asn Gln Ser Phe Ser Asn Gly Leu Ala Cys Leu Asp Met Val Leu Arg
        275                 280                 285

Ile Ala Asp Val Lys Glu Glu Asp Leu Leu Leu Gln Tyr Asp Cys Leu
    290                 295                 300

Ala Leu Asn Leu His Gly Leu Arg Arg His Thr Val Arg Leu Ser Arg
305                 310                 315                 320

Lys Asn Pro Ser Lys Glu Cys Phe
                325

<210>  4
<211>  220
<212>  PRT
<213>  Homo sapiens

<400>  4

Met Gly Ala Ala Ala Arg Thr Leu Arg Leu Ala Leu Gly Leu Leu Leu
1               5                   10                  15

Leu Ala Thr Leu Leu Arg Pro Ala Asp Ala Cys Ser Cys Ser Pro Val
            20                  25                  30

His Pro Gln Gln Ala Phe Cys Asn Ala Asp Val Val Ile Arg Ala Lys
        35                  40                  45

Ala Val Ser Glu Lys Glu Val Asp Ser Gly Asn Asp Ile Tyr Gly Asn
    50                  55                  60

Pro Ile Lys Arg Ile Gln Tyr Glu Ile Lys Gln Ile Lys Met Phe Lys
65                  70                  75                  80

Gly Pro Glu Lys Asp Ile Glu Phe Ile Tyr Thr Ala Pro Ser Ser Ala
                85                  90                  95

Val Cys Gly Val Ser Leu Asp Val Gly Gly Lys Lys Glu Tyr Leu Ile
            100                 105                 110

Ala Gly Lys Ala Glu Gly Asp Gly Lys Met His Ile Thr Leu Cys Asp
        115                 120                 125

Phe Ile Val Pro Trp Asp Thr Leu Ser Thr Thr Gln Lys Lys Ser Leu
    130                 135                 140

Asn His Arg Tyr Gln Met Gly Cys Glu Cys Lys Ile Thr Arg Cys Pro
145                 150                 155                 160

Met Ile Pro Cys Tyr Ile Ser Ser Pro Asp Glu Cys Leu Trp Met Asp
                165                 170                 175

Trp Val Thr Glu Lys Asn Ile Asn Gly His Gln Ala Lys Phe Phe Ala
            180                 185                 190

Cys Ile Lys Arg Ser Asp Gly Ser Cys Ala Trp Tyr Arg Gly Ala Ala
        195                 200                 205

Pro Pro Lys Gln Glu Phe Leu Asp Ile Glu Asp Pro
    210                 215                 220